# EUROPEAN PATENT APPLICATION

(11) **EP 4 847 534 A1**
(43) Date of publication of application: **02.12.2026**
(21) Application number: 25179628.0
(22) Date of filing: 28.05.2025
(51) Int. Cl.: C07K 16/32, C07K 14/31, C07K 14/315

(54) **IMMUNOGLOBULIN CONJUGATES**

(71) Applicant: MU Bioteknik Aktiebolag, 181 90 Lidingö (SE)
(72) Inventor: UHLÉN, Mathias, 181 90 Lidingö (SE); NYGREN, Per-Åke, 178 40 Ekerö (SE); DAHLSSON LEITAO, Charles, 117 69 Stockholm (SE)
(74) Representative: Kransell & Wennborg KB

(57) **Abstract**

There is provided a compound comprising at least one conjugation site, two immunoglobulin-binding moieties and a peptide linker having a length of 35-55 amino acids, wherein the immunoglobulin-binding moieties are connected by the peptide linker and bind to the Fc region of an immunoglobulin.

## Description

### TECHNICAL FIELD

The present disclosure relates to the field of immunoglobulin technology, in particularly to immunoglobulin conjugates.

### BACKGROUND

Immunoglobulins, i.e., antibodies, are widely used in therapeutic, diagnostic, and research applications due to their high specificity and affinity for target antigens. For many applications, the antibodies need to be labelled with e.g. fluorescent groups, enzymes or nucleic acids, to facilitate their detection and/or to provide handles for diagnostic assays

Antibody-binding proteins have been used in some antibody-based technologies wherein protein A from *Staphylococcus aureus,* protein G from streptococcal bacteria and protein L from *Peptostreptococcus magnus* are commonly used. However, achieving stable and specific interaction with antibodies, particularly without interfering with the antigen-binding site, remains a technical challenge.

### SUMMARY

Staphylococcal protein A and protein G, and especially their engineered derivatives, such as Z-domains and engineered C2 domains, are well known for their ability to bind the Fc region of immunoglobulin (Ig) without interfering with antigen recognition. The use of these engineered immunoglobulin-binding domains enables targeted binding to the Fc region while preserving immunoglobulin functionality. However, the affinity of monomeric immunoglobulin-binding domains may be insufficient for applications requiring high stability or prolonged binding.

The present inventors realized that by utilizing a compound comprising at least one conjugation site and two immunoglobulin-binding moieties connected by a peptide linker having an optimal length, the binding strength of the compound to an immunoglobulin can be significantly improved, and the dissociation rates can be reduced.

Accordingly, a compound is provided comprising at least one conjugation site, two immunoglobulin-binding moieties and a peptide linker having a length of 35-55 amino acids, wherein the immunoglobulin-binding moieties are connected by the peptide linker and bind to the Fc region of an immunoglobulin.

A compound according to the present disclosure is capable of binding to both symmetrical sites on the same Fc region of an immunoglobulin, i.e., each of the two immunoglobulin-binding moieties bind to the same Fc region of an immunoglobulin, resulting in enhanced binding strength. Furthermore, the optimized peptide linker length further improves the binding to the immunoglobulin while also allowing for a slower dissociation compared to peptide linkers having a longer or shorter peptide linker. Thus, this configuration significantly enhances the binding strength to an immunoglobulin while also reducing the dissociation rates. Additionally, the introduction of at least one conjugation site enables conjugation and thus functionalization of the immunoglobulin.

The conjugation site may be a cysteine residue or a lysine residue.

The compound may comprise one conjugation site, preferably one cysteine residue.

In another embodiment, the compound comprises two or more conjugation sites. In this embodiment, the conjugation site may be a cysteine residue or a lysine residue.

Preferably, the immunoglobulin-binding moieties are selected from Protein A-derived domains and Protein G-derived domains. The Protein A-derived domain may be a mutated or native B domain, preferably it is a mutated B domain.

In one embodiment, the mutated B domain comprises an amino acid sequence selected from SEQ ID No:1 and SEQ ID No:2 or an amino acid sequence which has at least 95% identity to SEQ ID No:1 or SEQ ID No:2. The amino acid sequence SEQ ID No:1 may be referred to as the Z-domain and the amino acid sequence SEQ ID No:2 may be referred to Z(Phe5IIIe).

The Protein G-derived domains may comprise an amino acid sequence SEQ ID No:3 or an amino acid sequence which has at least 95% identity to SEQ ID No:3.

The peptide linker preferably has a length of 37-50 amino acids, more preferably 38-48. This peptide linker length is associated with significantly reduced dissociation rates when the compound is bound to immunoglobulins.

Preferably, the peptide linker does not comprise two adjacent basic amino acids.

In one embodiment, at least 90% of the amino acids in the peptide linker are selected from alanine, glycine, asparagine, serine, threonine, isoleucine, leucine, methionine, valine, lysine, cysteine and/or glutamine, preferably at least 90% of the amino acids in the peptide linker are selected from alanine, glycine, asparagine, serine and/or threonine, more preferably at least 90% of the amino acids in the peptide linker are selected from glycine and serine.

The peptide linker may comprise at least 6 repeats of an amino acid sequence SEQ ID No:4 or an amino acid sequence which has at least 80% identity to SEQ ID No:4. The amino acid sequence SEQ ID No:4 may be referred to as G₄S.

In a preferred embodiment, the peptide linker comprises 7-11 repeats, preferably 8-9 repeats, of the amino acid sequence SEQ ID No:4 or an amino acid sequence which has at least 80% identity to SEQ ID No:4.

The compound may further comprise an amino acid sequence SEQ ID No:5, preferably the amino acid sequence SEQ ID No:5 is located N-terminal or C-terminal of one of the immunoglobulin-binding moieties. The amino acid sequence SEQ ID No:5 can also referred be to as a His₆-tag.

The compound may further comprise an amino acid sequence SEQ ID No:6, preferably the amino acid sequence SEQ ID No:6 is located N-terminal or C-terminal of one of the immunoglobulin-binding moieties. The amino acid sequence SEQ ID No:6 can also be referred to as an Avi-tag.

The compound may further comprise additional immunoglobulin-binding moieties that bind to the Fc region of an immunoglobulin. In this embodiment, the compound comprises more than two immunoglobulin-binding moieties that bind to the Fc region of immunoglobulins.

The compound may further comprise additional domains such as functional domains and albumin-binding domains. These additional domains may be located either C-terminal or N-terminal of one of the immunoglobulin-binding moieties.

The compound may be a fusion protein.

The compound further comprises a DNA sequence or a reporter molecule conjugated to the at least one conjugation site.

### BRIEF DESCRIPTION OF THE DRAWINGS

Aspects and embodiments are now described, by way of example, with reference to the accompanying drawings, in which:

Fig 1 shows a) a schematic overview of a compound binding to an immunoglobulin which in turn binds to an antigen. The compound comprises a DNA sequence conjugate or a reporter molecule conjugate; b) schematic structures of compounds according to the present disclosure. Zwt is a Z domain, Z(Phe5Ile) is the mutated Z domain, aa is amino acid, Avi is an Avi-tag, C is a cysteine, ABD is an albumin-binding domain, C2(N36Y) is the mutated C2 domain.

Fig 2 shows the SDS-PAGE of different Z-variants. Z-Gly-Z corresponds to ZZ, Z-45-Z corresponds to Z-(G₄S)₉-Z and monomeric Z corresponds to Z in Example 1. Zwt is a Z domain, aa is amino acid and C is a cysteine.

Fig 3 shows the sensorgrams from SPR for Z and ZZ in Example 1. The injected concentrations of the Z-variants were 400, 200, 100 and 50 nM. The immobilization levels of trastuzumab for the sensorgrams shown here is 500 response units (RU).

Fig 4 shows the sensorgrams from SPR for different peptide linker compositions and lengths in Example 1. The injected concentrations of the Z-variants were 400, 200, 100 and 50 nM. The immobilization levels of IgG1 (trastuzumab) for the sensorgrams shown here is 500 response units (RU).

Fig 5 shows the results from SDS-PAGE for different Z-variants in Example 1.

Fig 6 shows the sensorgrams from SPR when Z-(G₄S)₈-Z (Z-40-Z) binds to IgGs from different species. IgG1 - human IgG1, IgG2 - human IgG2, mIgG1 - mouse IgG1, mIgG2a - mouse IgG2a, mIgG2b - mouse IgG2b and Rabbit IgG. The concentrations injected were 100, 50, 25, 12.5 and 6.25 nM.

Fig 7 shows a) SDS-PAGE of Z, ZZ (Z-Z), Z-(G₄S)8-Z after purification using IMAC and after HRP-conjugation (Z-HRP, ZZ-HRP (Z-Z_HRP), Z-(G₄S)8-Z-HRP); b) ELISA results of HER2 detection using Z-HRP, ZZ-HRP (Z-Z_HRP), Z-(G₄S)8-Z-HRP.

### DETAILED DESCRIPTION

The ability to achieve a stable and specific interaction with immunoglobulins, without interfering with the antigen-binding site, is highly desired in immunoglobulin-based technologies. The present disclosure relates to a compound which may form a stable and specific interaction with immunoglobulins allowing for site-specific labelling, detection, or functionalization of immunoglobulins without chemical modification of the immunoglobulin itself.

Antibody and immunoglobulin are used interchangeably in this specification.

The compound according to the present disclosure comprises at least one conjugation site, two immunoglobulin-binding moieties and a peptide linker having a length of 35-55 amino acids, wherein the immunoglobulin-binding moieties are connected by the peptide linker and bind to the Fc region of an immunoglobulin.

The compound according to the present disclosure allows for a strong binding to an immunoglobulin and a reduced dissociation rate. This is enabled by combining two immunoglobulin-binding moieties with a flexible peptide linker. Furthermore, this compound allows for a modular and non-disruptive strategy for immunoglobulin functionalization and detection, suitable for diverse biotechnological and biomedical applications. The functionalization is enabled by the introduction of the at least one conjugation site.

The immunoglobulin-binding moieties may be connected to the peptide linker via a covalent or non-covalent bond, preferably via a covalent bond. When the immunoglobulin-binding moieties are covalently bound to the peptide linker, the compound is preferably a fusion protein.

The immunoglobulin-binding moieties bind to the Fc-region of an immunoglobulin, preferably to the heavy chain region of the Fc region, more preferably between CH2 and CH3-domains of the heavy chain. Binding to the Fc region of an immunoglobulin offers several advantages for immunoglobulin-targeted applications. The Fc region provides an accessible site on immunoglobulins. Furthermore, binding to the Fc region does not interfere with the antigen-binding function of the immunoglobulin, allowing the immunoglobulin to retain its specificity and activity.

Each of the two immunoglobulin-binding moieties preferably bind to each of the symmetrical sites on the same Fc region of an immunoglobulin. More preferably, each of the immunoglobulin-binding moieties bind to each of the two identical heavy chains of the Fc region.

The immunoglobulin-binding moieties preferably bind to the Fc region of immunoglobulin G (IgG).

The monovalent affinity of an immunoglobulin-binding moiety for the Fc region of immunoglobulins is relatively weak (typically 10-30 nM). By fusing two immunoglobulin-binding moieties together using a sufficiently long linker, the apparent affinity can be drastically improved due to strong avidity effects caused by the binding to each of the two identical heavy chains of the Fc region.

Preferably, the two immunoglobulin-binding moieties comprise the same amino acid sequence.

The immunoglobulin-binding moieties are selected from Protein A-derived domains and Protein G-derived domains.

Protein A comprises five homologous immunoglobulin-binding moieties called domain E, domain D, domain A, domain B and domain C.

Preferably, the immunoglobulin-binding moiety of the present disclosure is a mutated or native B domain. The B domain has 58 amino acid residues. In a specific mutated version of domain B referred to as domain Z (SEQ ID No:1), the alanine (Ala) residue in position 1 is replaced with valine (Val) and the glycine (Gly) residue in position 29 is replaced with an Ala residue. The Gly29Ala mutation reduces the Fab-binding capability of the native B domain. It is desired to reduce the Fab-binding so not to reduce the antigen binding capability. The Ala1Val mutation is optional.

In a preferred embodiment, the mutated B domains comprise the amino acid sequence SEQ ID No:1 or an amino acid sequence which has at least 95% identity to SEQ ID No:1. Thus, the mutated B domain may be the Z domain.

In another embodiment, the immunoglobulin-binding domain comprises the amino acid sequence SEQ ID No:2 or an amino acid sequence which has at least 95% identity to SEQ ID No:2. This is a mutated Z domain wherein the phenylalanine (Phe) at position 5 is replaced with Isoleucine (Ile). This Phe4Ile mutation may allow improved binding to mouse IgG.

In a further embodiment, the immunoglobulin-binding moiety is a Protein G-derived domain.

Protein G is derived from *Streptococcus* species and is known for its immunoglobulin-binding ability. Protein G comprises multiple immunoglobulin-binding moieties, typically referred to as C1, C2, and C3 domains, each capable of binding to the Fc region of immunoglobulins. The C2 domain consists of approximately 55 amino acid residues. The N36Y mutation (substituting asparagine (Asn) at position 36 with tyrosine (Tyr)) of C2 domain enables the elimination of Fab-binding without significantly impairing Fc-binding.

In one embodiment, the Protein G-derived domain comprises the amino acid sequence SEQ ID No:3 or an amino acid sequence which has at least 95% identity to SEQ ID No:3.

The peptide linker has a length of 35-55 amino acids. This length enables a particularly good avidity effect combined with a significantly reduced dissociation. Preferably, the peptide linker has a length of 37-50 amino acids, more preferably 38-48. Using compounds comprising these peptide linker lengths may result in a further reduction the dissociation rates.

The peptide linker may comprise any amino acid, in any configuration. However, it may be preferred to not include two adjacent basic amino acids as this may cause instability and degradation of the peptide linker. The basic amino acids are e.g. lysine and arginine.

Furthermore, it is preferred that the peptide linker is flexible which enables improved binding to the immunoglobulin as well as reduces the dissociation rates.

In one embodiment, at least 90% of the amino acids in the peptide linker are selected from alanine, glycine, asparagine, serine, threonine, isoleucine, leucine, methionine, valine, lysine, cysteine and/or glutamine, preferably at least 90% of the amino acids in the peptide linker are selected from alanine, glycine, asparagine, serine and/or threonine, more preferably at least 90% of the amino acids in the peptide linker are selected from glycine and serine. These amino acids may give rise to an especially flexible and stable linker peptide linker.

In one embodiment, the peptide linker comprises at least 6 repeats of an amino acid sequence SEQ ID No:4 or an amino acid sequence which has at least 80% identity to SEQ ID No:4. This amino acid sequence gives rise to an especially flexible and stable peptide linker. Using glycine in the peptide linker may significantly improve the flexibility of the peptide linker while using serine may improve solubility and stability due to its polar and hydrophilic nature.

In a preferred embodiment, the compound comprises a peptide linker comprises 7-11 repeats, preferably 8-9 repeats, of the amino acid sequence SEQ ID No:4 or an amino acid sequence which has at least 80% identity to SEQ ID No:4.

The compound comprises at least one conjugation site. The at least one conjugation site may be part of the linker, part of the immunoglobulin-binding moieties and/or located-terminal or C-terminal of one of the immunoglobulin-binding moieties.

The conjugation site may comprise a functional group which allows for conjugation. The conjugation site may be a functional residue, i.e., a residue having a suitable side chain enabling conjugation.

The at least one conjugation site may be a cysteine residue or a lysine residue, preferably it is a cysteine.

In one embodiment, the compound comprises one conjugation site. In this embodiment, the conjugation site is preferably a cysteine.

In another embodiment, the compound comprises two or more conjugation sites.

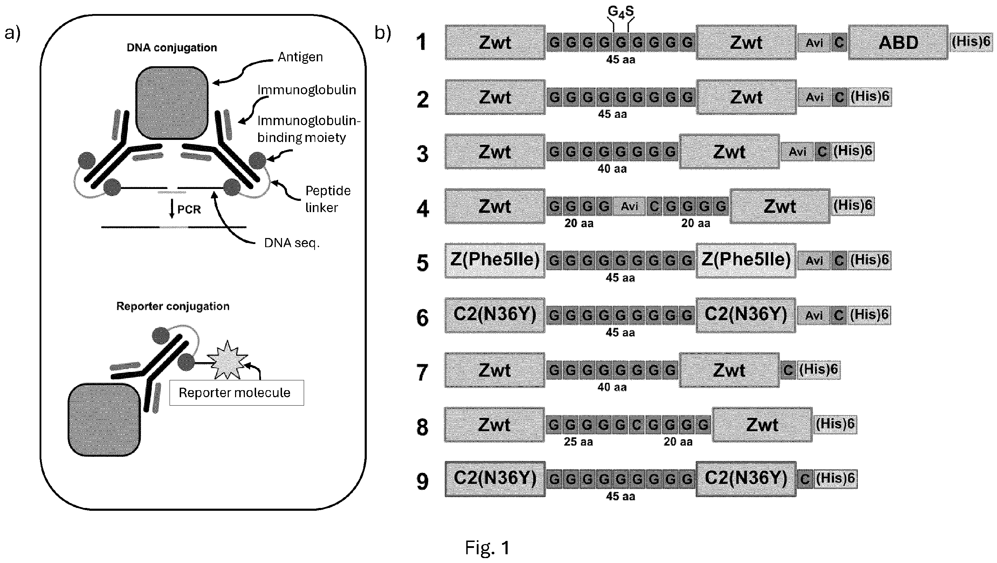

The compound may further comprise a DNA sequence or a reporter molecule conjugated to at least one conjugation site. By functionalizing the compound with a DNA sequence or a reporter molecule, it can be used to study immunoglobulin interactions without directly modifying the immunoglobulin. An example of a reporter molecule is maleimide-Horseradish Peroxidase (maleimide-HRP). Fig. 1a shows a compound conjugated with a DNA sequence or a reporter molecule binding to an immunoglobulin which in turn binds to an antigen.

When a DNA sequence is to be conjugated, the compound preferably only comprises one cysteine residue. When a reporter molecule is to be conjugated, the compound preferably comprises two or more conjugation sites. Having more than one conjugation site allows for more reporter molecules to be conjugated and thus an improved signal may be obtained.

Conjugation to the lysine residue may be performed by using NHS-ester functionalized molecules which have the ability to react with the e-amino group of lysine residues to form stable amide bonds.

The compound may comprise at least one cysteine residue, preferably the compound comprises one cysteine residue, which may be positioned at any location within the compound. Preferably, the cysteine residue is located N-terminal or C-terminal of one of the immunoglobulin-binding moieties.

The compound of the present disclosure may further comprise additional moieties which allow for e.g. purification or functionalization. The additional moieties may be incorporated into the peptide linker or located N-terminal or C-terminal of one of the immunoglobulin-binding moieties. Preferably, the additional moieties are located C-terminal of one of the immunoglobulin-binding moieties. Incorporating these moieties into the compound enables site-specific labelling, detection, or functionalization of immunoglobulins without chemical modification of the immunoglobulin itself.

The compound may comprise additional moieties to facilitate the purification of the compound. The compound may e.g. comprise an amino acid sequence SEQ ID No:5, preferably the amino acid sequence SEQ ID No:5 is located N-terminal or C-terminal of one of the immunoglobulin-binding moieties. This specific amino acid sequence allows for purification using immobilized metal ion affinity chromatography (IMAC). IMAC is a straightforward and fast purification method, often resulting in high purity.

The compound may further comprise an amino acid sequence SEQ ID No:6, preferably the amino acid sequence SEQ ID No:6 is located N-terminal or C-terminal of one of the immunoglobulin-binding moieties. This amino acid sequence enables site-specific biotinylation.

The compound may further comprise additional immunoglobulin-binding moieties that bind to the Fc region of an immunoglobulin. In this embodiment, the compound comprises more than two immunoglobulin-binding moieties that bind to the Fc region of immunoglobulins. The embodiments concerning the two immunoglobulin-binding moieties apply *mutatis mutandis* to the additional immunoglobulin-binding moieties.

The compound may further comprise additional domains such as functional domains and albumin-binding domains. These additional domains may be located either C-terminal or N-terminal of one of the immunoglobulin-binding domains.

In one embodiment, the at least one conjugation site is located on the functional domain. The functional domain may comprise an amino acid sequence selected from SEQ ID No:1 and SEQ ID No:2 or an amino acid sequence which has at least 95% identity to SEQ ID No:1 or SEQ ID No:2.

In a most preferred embodiment, the compound comprises a peptide linker comprising 7-11 repeats, preferably 8-9 repeats, of the amino acid sequence SEQ ID No:4 or an amino acid sequence which has at least 80% identity to SEQ ID No:4 and further comprises the amino acid SEQ ID No:1 or the amino acid sequence which has at least 95% identity to SEQ ID No:1 as the immunoglobulin-binding moieties. This specific compound preferably comprises cysteine as the at least one conjugation site and/or comprises the amino acid sequence SEQ ID No:5.

The term "% identity", as used throughout the specification, is calculated as follows. The query sequence is aligned to the target sequence using the CLUSTAL W algorithm (Thompson et al (1994) Nucleic Acids Research, 22:4673-4680). A comparison is made over the window corresponding to the target sequence. The amino acid residues at each position are compared and the percentage of positions in the query sequence that have identical correspondences in the target sequence is reported as % identity.

The compounds of the present disclosure can be produced efficiently in *E*. *coli,* purified via IMAC, and exhibit high solubility and stability. Binding assays, including ELISA and SPR, demonstrate that compound having a long flexible linker between two immunoglobulin-binding moieties exhibit superior binding performance to various human and mouse immunoglobulin isotypes.

Fig 1b shows schematic examples of different compounds according to the present disclosure.

### EXAMPLES

### Example 1

In this example a panel of compounds comprising the Z-domain (SEQ ID No:1) as the immunoglobulin-binding moiety was used to evaluate the effect of linker length and composition on avidity-driven binding strength to the Fc region of immunoglobulins. The prepared compounds, i.e., Z-variants, include a monomeric Z-variant (Z), a dimeric variant with a single glycine between the immunoglobulin-binding moieties (ZZ) and dimeric variants where the immunoglobulin-binding moieties are interconnected by a peptide linker of various sizes and compositions. Peptide linkers comprising either G₄S-segments or DDAKK-segments and having between 20 and 45 amino acids were prepared, see Table 1. All Z-variants contain a C-terminal His₆-tag (SEQ ID No:5) to allow for purification using IMAC. Additional Z-variants containing C-terminal cysteine residues (Z-C, ZZ-C and Z(G₄S)₈Z-C) were also included to enable site-specific functionalization. All the prepared Z-variants can be seen in Table 1.

Table 1 shows the Z-variants prepared in Example 1. All Z-variants were functionalized with a His₆-tag.

| **Compound** | **Peptide linker composition*** | **Linker length** | **Cysteine** |
|---|---|---|---|
| Z | - | - | No |
| ZZ | G | 1 | No |
| Z-(G₄S)₄-Z | G₄S | 20 | No |
| Z-(G₄S)₆-Z | G₄S | 30 | No |
| Z-(G₄S)₈-Z | G₄S | 40 | No |
| Z-(G₄S)₉-Z | G₄S | 45 | No |
| Z-(DDAKK)₄-Z | DDAKK | 20 | No |
| Z-(DDAKK)₆-Z | DDAKK | 30 | No |
| Z-C | - | - | Yes |
| ZZ-C | G | 1 | Yes |
| Z-(G₄S)₈-Z-C | G₄S | 40 | Yes |

| | | | |
|---|---|---|---|
| *This is the repeating segment of the peptide linker. i.e., for Z-(G₄S)₈-Z there are 8 G₄S segments in the peptide linker. | | | |

### Materials and Methods

### Production and purification of Z-variants

The genes for all Z-variants were synthesized and cloned into a pET29b(+) vector by Twist Bioscience. *Escherichia coli* BL21(DE3)* cells were transformed with the plasmids using standard heat-shock treatment. A single colony for each variant was used to inoculate 10 mL of TSB-Y media supplemented with 50 ng/mL of kanamycin (Kan [50]) and incubated overnight. After 16 h, the O/N cultures were diluted 1:100 in TSB-Y supplemented with Kan [50] to a final volume of 500 mL. The cultures were induced with IPTG to a final concentration of 1 mM at OD₆₀₀ ≈ 0.7 and incubated overnight at 27 °C and 150 rpm. After 16 h, the cells were harvested by centrifugation for 10 min at 5000xg. The harvested cells were lysed by sonication for 1.5 min (1s ON/1s OFF) followed by centrifugation at 4 °C and 10000xg for 20 min. The supernatant was filtered (0.45 µm) and the variants were purified using IMAC. PD-10 desalting columns packed with 3 mL TALON Metal Affinity Resin, wash buffer (50 mM Na₂HPO₄, 500 mM NaCl, 20 mM imidazole, pH 8) and elution buffer (50 mM Na₂HPO₄, 500 mM NaCl, 500 mM imidazole, pH 8) were used according to manufacturer's instructions (Cytiva, Sweden). Following elution from the IMAC column, the buffer was changed to PBS using PD-10 desalting columns according to manufacturer's instructions (Cytiva, Sweden). The purified Z-variants were analysed by mass spectrometry (4800 MALDI TOF/TOF, Applied Biosystems, USA) and SDS-page gels (NuPAGE, Invitrogen, USA).

### Functionalization of Z-variants with HRP and binding evaluation using ELISA

Each Z-variant with a cysteine was diluted to a concentration of 25 µM in PBS (pH 7.2). Freshly dissolved tris(2-carboxyethyl)phosphine (TCEP) was added at a tenfold molar excess of 250 µM and incubated for 30 min at 37 °C. EZ-Link Maleimide Activated Horseradish Peroxidase (Thermo Scientific) was added to a final concentration corresponding to four times molar excess relative to the protein concentration which was diluted to 12.5 µM and incubated at room temperature overnight. The success and degree of conjugation was evaluated using MALDI-TOF MS (4800 MALDI TOF/TOF, Applied Biosystems, USA).

The HRP-conjugated Z-variants were evaluated using enzyme-linked immunosorbent assay (ELISA). A high-binding 96-well plate (Corning) was coated at 4°C overnight with a 1:10 dilution series of HER2 (His-tag, Acro Biosystems) ranging from 10 nM to 1 pM in 50 mM carbonate buffer (pH 9.4) in duplicates for each Z-variant (Z-HRP, ZZ-HRP and Z-(G₄S)₈-Z-HRP). The wells were then aspirated and washed with phosphate-buffered saline supplemented with 0.05% Tween20 (PBST). Blocking buffer (PBST supplemented with 3% bovine serum albumin) was added to each well and incubated for 1 hour at room temperature. The anti-HER2 antibody trastuzumab (Testa Center, Sweden) was added to each well at a concentration of 2.6 ug/mL and incubated at room temperature for 1 hour with gentle shaking (300 RPM). After washing the wells five times with PBST, the HRP-conjugated Z-variants were added at a concentration of 100 nM to their respective wells and incubated for 90 min at room temperature with gentle shaking. The wells were aspirated and washed five times with PBST followed by incubation in PBST for 45 min at room temperature. 3,3',5,5'-tetramethylbenzidine (TMB) substrate solution was added to each well and incubated for approximately 20 min. The reaction was stopped using 0.2 M H₂SO₄ and the absorbance at 450 nm was measured.

### Evaluation of avidity-driven binding strength using surface plasmon resonance

The effect of peptide linker composition and length on avidity driven binding strength was evaluated using surface plasmon resonance (SPR) on a Biacore 8K instrument. Trastuzumab, a HER2-binding IgG1, was immobilized at relatively low levels of both 500 and 1000 response units (RU) on a CM5 chip, corresponding to a theoretical RUₘₐₓ of approximately 25-50 and 50-100 RU, respectively. This was done to limit intermolecular avidity effects, i.e. binding to two separate IgG1 molecules on the surface and instead favor avidity effects from binding to the Fc region of the same IgG1 molecule. The Z-variants were injected at concentrations of 400, 200, 100 and 50 nM over the IgG1 surface over 120 seconds and allowed to dissociate over 1200s, using a flow rate of 30 µL/min. The surfaces were regenerated by injection of 10 mM HCl over 30 seconds.

The dissociation rates of the Z-variants binding to Trastuzumab were estimated from sensorgrams using the Biacore 8K evaluation software, only fitting the dissociation rates with a starting value of 10⁻².

### Results

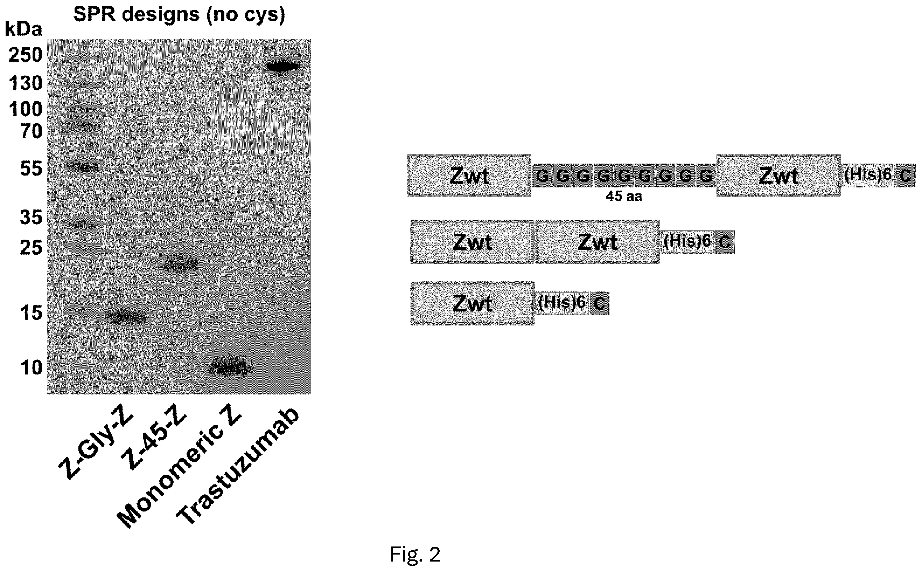

The prepared Z-variants were characterized by SDS-PAGE, see Fig. 2, which also shows the schematic structure of some of the Z-variants.

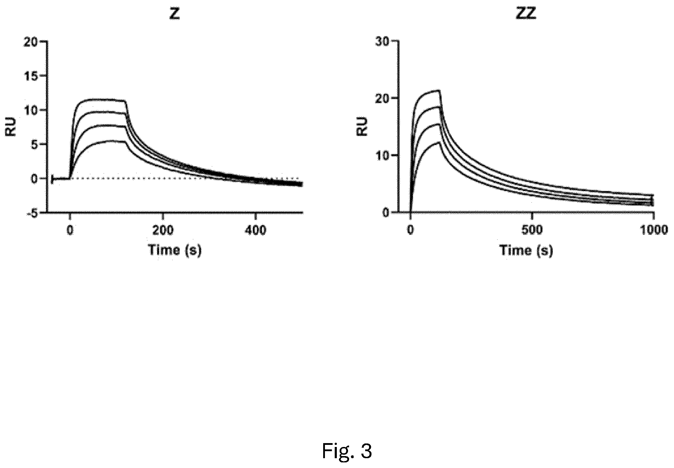

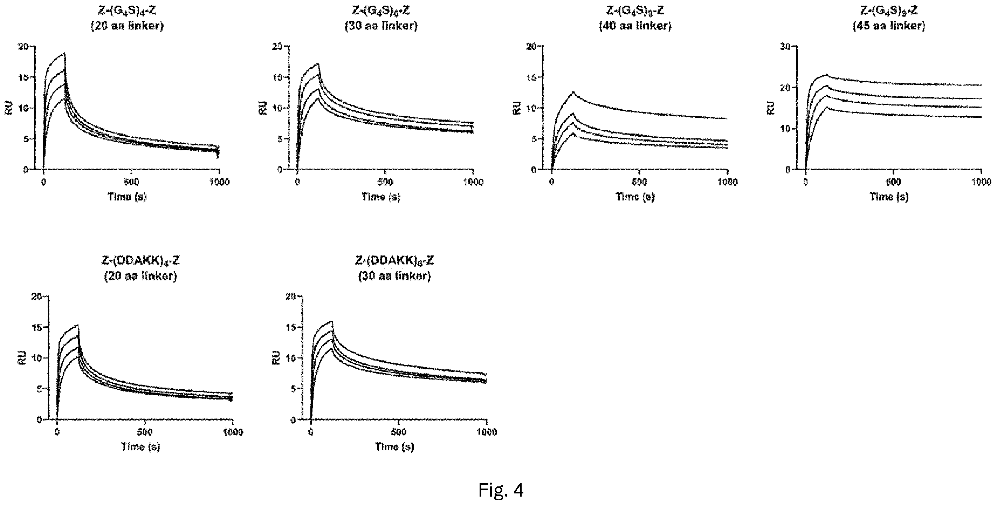

The effect of peptide linker composition and length on avidity driven binding strength was evaluated using SPR and the results are shown in Fig. 3-4. The immobilization level of trastuzumab (human IgG1) was 500 RU.

The results clearly show that by increasing the length of the peptide linker, increased avidity effects and slower dissociation rates are obtained. The increased avidity effects and the reduced dissociation rates were independent of the peptide linker composition, indicating the significance of the length of the peptide linker. The avidity effects were most pronounced for Z-(G₄S)₉-Z which has a 45 amino acid long peptide linker. This improvement in avidity-driven binding can be discerned qualitatively from the sensorgrams obtained from SPR but has also been quantitatively estimated from approximations of the dissociation rates, see Table 2.

Table 2 shows the dissociation rates of Z-variants binding to Trastuzumab estimated from the sensorgrams in Fig. 3-4. "1/kd" values are meant to facilitate comparison of dissociation rates, with higher values corresponding to slower dissociation rates and stronger avidity-driven binding.

| **Compound** | **Linker length** | **kd (1/s)** | **1/kd** |
|---|---|---|---|
| Z | - | 7.30x10⁻³ | 137 |
| ZZ | 1 | 8.07x10⁻³ | 124 |
| Z-(G₄S)₄-Z | 20 | 1.68x10⁻² | 60 |
| Z-(G₄S)₆-Z | 30 | 3.45x10⁻⁴ | 2895 |
| Z-(G₄S)₈-Z | 40 | 2.98x10⁻⁴ | 3352 |
| Z-(G₄S)₉-Z | 45 | 8.17x10⁻⁵ | 12238 |
| Z-(DDAKK)₄-Z | 20 | 1.68x10⁻² | 59 |
| Z-(DDAKK)₆-Z | 30 | 3.41x10⁻⁴ | 2931 |

Table 2 shows that the dissociation rate reduces with increased length of the peptide linker, with a three-fold reduction of the dissociation rate when increasing the peptide linker length from 40 to 45 amino acids.

The level of immobilization of trastuzumab was increased from 500 RU to 1000 RU. The increase led to an increase in the response levels of the injected Z-variants, but the kinetics remained similar for all Z-variants. This indicates that the avidity effects are intramolecular rather than intermolecular.

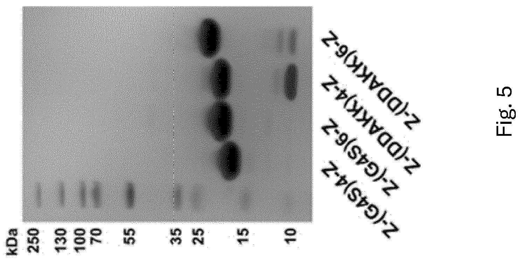

SDS-PAGE was conducted on Z-(G₄S)₄-Z, Z-(G₄S)₆-Z, Z-(DDAKK)₄-Z and Z-(DDAKK)₆-Z (i.e., Z-variants having peptide linker lengths of 20 or 30 amino acids) following IMAC purification, see Fig. 5 Degradation products can be seen for the Z-variants comprising DDAKK linkers. This is most likely due to lysine-mediated proteolysis causing degradation. The Z-variants comprising G₄S linkers do not exhibit any degradation and are thus more stable.

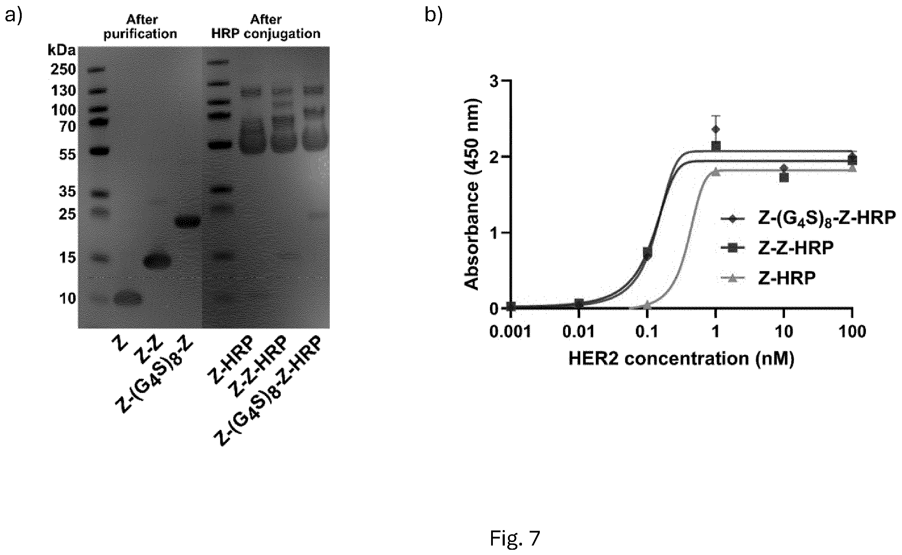

The Z-variants comprising cysteine i.e., Z-C, ZZ-C and Z-(G₄S)₈-Z-C, were functionalized with HRP to form Z-HRP, ZZ-HRP and Z-(G₄S)₈-Z-HRP. SDS-PAGE shows the bands corresponding to Z-HRP, ZZ-HRP and Z-(G₄S)₈-Z-HRP following IMAC purification and HRP conjugation, see Fig. 7a. As can be seen in Fig. 7a, each variant has been conjugated to HRP almost fully. The strong band around 55 kDa is unreacted excess of maleimide-HRP.

The HRP-functionalized Z-variants ability to detect HER2 by binding to Trastuzumab was evaluated, see Fig. 7b. It was observed that all tested HRP-functionalized Z-variants can detect HER2 by binding to Trastuzumab. These results further show that Z-variants comprising two antibody-binding moieties can detect HER2 at lower concentrations than the Z-variant comprising only one antibody-binding moiety. A difference in signal sensitivity of a factor ten between monovalent and bivalent variants was observed. No significant difference in detection could be observed between ZZ-HRP and Z-(G₄S)₈-Z-HRP.

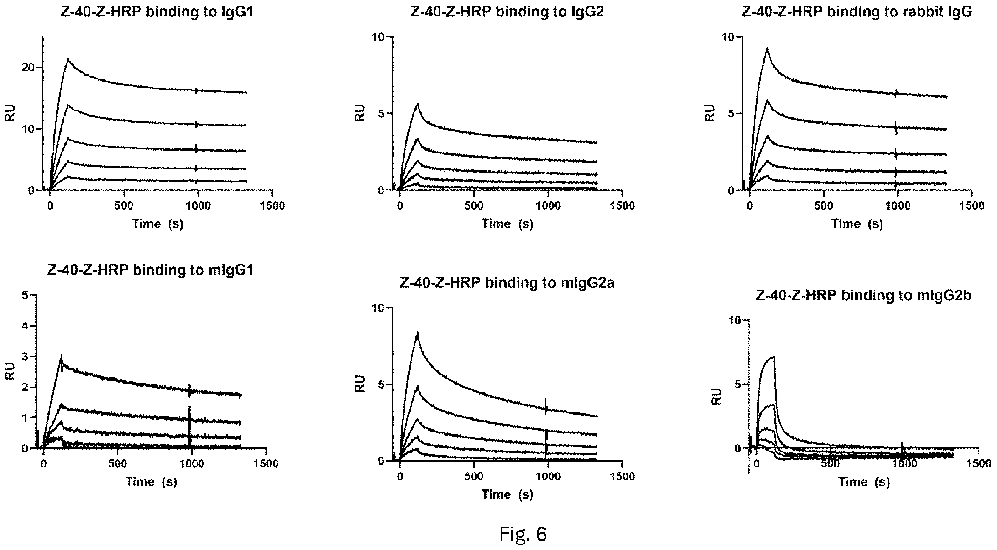

Furthermore, the binding of HRP functionalized Z-(G₄S)₆-Z (Z-(G₄S)₆-Z - HRP) to IgG of different species were evaluated, see Fig. 6. The different species were human IgG1 (Trastuzumab), human IgG2 (Seribantumab), mouse IgG1, mouse IgG2a, mouse IgG2b and rabbit IgG. As can be seen, Z-(G₄S)₆-Z -HRP exhibits high avidity to most species as well as slow dissociation rates.

## Claims

1. A compound comprising at least one conjugation site, two immunoglobulin-binding moieties and a peptide linker having a length of 35-55 amino acids, wherein the immunoglobulin-binding moieties are connected by the peptide linker and bind to the Fc region of an immunoglobulin.

2. The compound according to claim 1, wherein the at least one conjugation site is a cysteine residue or a lysine residue.

3. The compound according to claim 2, wherein the compound comprises one conjugation site, preferably one cysteine residue.

4. The compound according to claim 2, wherein the compound comprises two or more conjugation sites.

5. The compound according to any one of the preceding claims, wherein the immunoglobulin-binding moieties are selected from Protein A-derived domains and Protein G-derived domains.

6. The compound according to claim 5, wherein the Protein A-derived domains are mutated or native B-domains.

7. The compound according to claim 6, wherein the mutated B domain comprises an amino acid sequence selected from SEQ ID No:1 and SEQ ID No:2 or an amino acid sequence which has at least 95% identity to SEQ ID No:1 or SEQ ID No:2.

8. The compound according to claim 5, wherein the Protein G-derived domain comprises an amino acid sequence SEQ ID No:3 or an amino acid sequence which has at least 95% identity to SEQ ID No:3.

9. The compound according to any one of the proceeding claims, wherein the peptide linker has a length of 37-50 amino acids, preferably 38-48 amino acids.

10. The compound according to any one of the preceding claims wherein at least 90 % of the amino acids in the peptide linker are selected from alanine, glycine, asparagine, serine, threonine, isoleucine, leucine, methionine, valine, lysine, cysteine and/or glutamine, preferably at least 90% of the amino acids in the peptide linker are selected from alanine, glycine, asparagine, serine, lysine, cysteine and/or threonine, more preferably at least 90% of the amino acids in the peptide linker are selected from glycine and serine.

11. The compound according to claim 10, wherein the peptide linker comprises at least 6 repeats of an amino acid sequence SEQ ID No:4 or an amino acid sequence which has at least 80% identity to SEQ ID No:4.

12. The compound according to claim 11, wherein the peptide linker comprises 7-11 repeats, preferably 8-9 repeats, of the amino acid sequence SEQ ID No:4 or the amino acid sequence which has at least 80% identity to SEQ ID No:4.

13. The compound according to any one of the proceeding claims, wherein the compound further comprises an amino acid sequence SEQ ID No:5, an amino acid sequence SEQ ID No:6 and/or an albumin-binding domain, preferably the amino acid sequence SEQ ID No:5, the amino acid sequence SEQ ID No:6 and/or the albumin-binding domain are located N-terminal or C-terminal of one of the immunoglobulin-binding moieties.

14. The compound according to any one of the preceding claims, wherein the compound is a fusion protein.

15. The compound according to any one of claims 1-13, wherein the compound further comprises a DNA sequence or a reporter molecule conjugated to the at least one conjugation site.
